# EUROPEAN PATENT APPLICATION

(11) **EP 0 692 253 A1**
(43) Date of publication of application: **17.01.1996**
(21) Application number: 94905842.4
(22) Date of filing: 03.02.1994
(51) Int. Cl.: A61K 31/73, A61K 31/14

(54) **PROPHYLATIC FOR DOMESTIC ANIMAL MASTITIS**

(71) Applicant: NIPPON SODA CO., LTD., Chiyoda-ku, Tokyo 100 (JP)
(72) Inventor: SAITO, Shigeru, R & D Lab. for Specialty Chemicals, Ichihara-shi, Chiba 290 (JP); TOMITA, Megumi, R & D Lab. for Specialty Chemicals, Ichihara-shi, Chiba 290 (JP)
(74) Representative: de Bruijn, Leendert C.
(86) International application number: JP9400160
(87) International publication number: WO9520968

(57) **Abstract**

A prophylactic for mastitis comprising a combination of chitosan or a derivative thereof with a quaternary ammonium salt bactericide. It has a potent bactericidal activity and can form a good chitosan film, and hence can prevent mastitis effectively. Since it does not irritate the skin so much, it is less liable to cause heretofore problematic cracking of the nipple due to the use of a medicine.

## Description

### Technical Field

The present invention relates to a mastitis preventive for preventing mastitis of livestock. More particularly, the invention relates to a preventive for mastitis characterized in that the preventive forms a coating comprising quaternary ammonium salt-containing sterilizing microbe with antibacterial and sterilizing property and chitosan and/or chitosan derivative on the udder of livestock such as dairy cattle.

### Background Art

The mastitis of livestock such as dairy cattle is a disease which causes inflammation in the udder due to the invasion of pathogenic bacteria, such as Staphylococcus aureus, Streptococcus agalactiae, Escherichia coli, etc. from the opening of teat into udder.

This disease is considered as serious disease particularly for dairy farmers and breaks out in high frequency. It gives them great loss, such as decrease of milk production of animals, degradation of milk quality, shortening of productive period of milk, hygienic problems due to the contamination of bacteria into milk and decrease of yield at processing for dairy products due to the increase of contamination of somatic cells in the milk.

Several means for preventing mastitis, for example, reinforcement of resistance of livestock against pathogenic microbes by breeding and maintaining them under an appropriate condition, milking under clean condition, sterilization of teats by dipping them into the solution containing sterilizing microbe, such as popidon iodine (PVP iodine), iodohole, chlorohexidine gluconate and benzalkonium, have been known among the dairy farmers.

It is known that there is another type of preventive for mastitis, which forms coating onto the udder for blocking the pathogenic microbes existing in outer environment from the teat of livestock. However, such coating itself does not have sterilizing activity therefore, it is not possible to prevent the infection of the pathogenic microbes into the udder during milking procedures.

### Disclosure of Invention

### (Problems in the Prior Arts)

The outbreaking ratio of mastitis can be reduced by the application of an drug microbe for dipping animal teats, however, the effectiveness of the microbe will vary depending upon the conditions, such as sterilizing activity and sterilizing rate of the microbe, bacterial species to be sterilized and long-lasting effectiveness of the microbe.

Whereas, it is also a serious point that the microbe causes no injuries on skin and mucosa of dairy cattle and human, despite of its sterilizing effectiveness.

The microbes for dipping animal teats containing iodine-containing sterilizing microbe such as popidon iodine have been widely used, however, they have strong stimulating action to skin and mucosa so that they cause rough skin and cracks in the teats of dairy cattle and human skin. A sterilizing microbe comprising chlorhexidine gluconate or the analogs has defects, such as slow sterilizing activity and strong stimulating action against mucosa. Another sterilizing microbe comprising benzalkonium chloride or the analogs has an advantage of less skin irritation, but it is less effective to some of the species in association with livestock mastitis. Furthermore, since these sterilizing microbes have no coating-forming property and are readily washed away, the sterilizing effectiveness thereof cannot be retained for a long time. For these reasons as described above, a preventive for mastitis which possesses strong sterilizing activity but no stimulating action to skin, and forms stable coating capable of preventing the infections of pathogenic microbes for a long time, has been required.

### (Problems to be Solved by the Invention)

It is an object of the present invention to provide a novel preventive for mastitis which forms coating onto the teats of livestock such as dairy cattle, prevents the propagation of the pathogenic microbes on the teats, inhibits the invasion of the pathogenic microbes into the udder, and has less skin irritation property.

### (Detailed Description of the Invention)

For solving the problems as described above, the inventors herein have made investigation and found that the combination of chitosan or chitosan derivatives with a quaternary ammonium salt-containing sterilizer has strong sterilizing activity and less stimulating action, and can inhibit the invasion of pathogenic microbes into the udder by forming an appropriate coating thereon.

Now, the present invention is further described hereinbelow in detail.

The preventive for mastitis of the present invention is characterized in that it is composed of a quaternary ammonium salt-containing sterilizing microbe and chitosan or a chitosan derivative as the active components.

Up till now, chitosan and its derivatives have been used in the fields, such as pharmaceuticals, cosmetics, industries, agriculture, food industries, etc. since they have various functional capabilities, such as antibacterial, sterilizing, wound healing and immune activating activities, and form a coating having high biological conformability.

The inventors herein noted the said excellent features of chitosan and its derivatives and found that the spraying or the dipping with the aqueous solution or acidic aqueous solution of chitosan or chitosan derivative is highly effective for the prevention of mastitis of livestock such as dairy cattle, then it was previously disclosed in Japanese Patent Application Serial No. Hei 2-290802. In addition thereto, the inventors herein also found the fact that a sterilizing microbe, benzalkonium chloride can be used together with chitosan or the derivatives thereof and their combination shows a synergistic activity for the prevention of mastitis. The present invention, Japanese Patent Application Serial No. Hei 4-233032, Filed on August 7, 1992, is directed to the fact as described immediately above.

Chitosan which may be used in the present invention is a deacetylated chitin, polysaccharide of N-acetyl-D-glucosamine, which is constitutional component of shells of crustaceans such as a crab and insects such as a beetle, and is basic polysaccharide high molecular weight substance, of which molecular weight is not particularly limited.

Chitin which is not deacetylated and the one containing the lightly-degraded products by chemical treatment can be used as well.

For the examples of chitosan derivatives, the ones wherein free amino groups or hydroxyl groups of chitosan are entirely or partially substituted by alkyl, alkoxyalkyl, alkylthioalkyl, aryl, aralkyl, acyl, sulfonyl, alkoxycarbonyl, phosphoric acid residue or the like are exemplified.

For example, these chitosan derivatives can be prepared by chemical modifications according to the methods commonly known in organic synthetic chemistry as described in the references hereinbelow.
(1) Japanese Patent Laid-opened No. Sho 57-180602
(2) Japanese Patent Laid-opened No. Sho 59-106409
The preventive for mastitis of the present invention comprising quaternary ammonium salt-containing sterilizing microbe and chitosan and/or the derivative thereof contains quaternary ammonium salt-containing sterilizing microbe in an amount of of from 0.2 to 100 parts by weight, preferably from 2 to 50 parts by weight, with respect to 100 parts by weight of chitosan and/or the derivative thereof.

The preventive for mastitis of the present invention is preferably used in aqueous solution or diluted aqueous solution of an organic acid or an inorganic acid containing quaternary ammonium salt-containing sterilizing microbe and chitosan and/or the derivative thereof at a concentration ranging from 0.05% by weight to 10% by weight.

For acids which may be used in the present invention, inorganic acids, such as hydrochloric acid and nitric acid and organic acids, such as acetic acid, citric acid, lactic acid, malic acid, etc are exemplified.

The dose of the preventive for mastitis to be applied will depends on the treatment process and the concentration of the preparation and is not particularly limited, however, it may be appropriately selected within the range which can demonstrate the preventive effects on mastitis.

For the quaternary ammonium salt-containing sterilizing microbes may be used in the present invention, benzalkonium chloride, benzethonium chloride (C₂₇H₄₂ClNO₂), cetyltrimethylammonium chloride, didecyldimethylammonium chloride, etc. are exemplified. Benzalkonium chloride herein is defined as a quaternary ammonium salt represented by the general formula, [C₆H₅CH₂N(CH₃)₂R]⁺Cl ⁻ wherein R represents straight chain or branched alkyl having 8 to 18 carbon atoms.

For the representative examples of the compounds according to the present invention, octyldimethylbenzylammonium chloride, decyldimethylbenzylammonium chloride, dodecyldimethylbenzylammonium chloride, tetradecyldimethylbenzylammonium chloride, hexadecyldimethylbe nzylammonium chloride, octadecylmethylbenzylammonium chloride, coconut alkyldimethylbenzylammonium chloride, etc. are exemplified.

The prevention of mastitis with the prepared preventive for mastitis comprising quaternary ammonium salt-containing sterilizing microbe and chitosan and/or the derivative thereof can be accomplished by spraying of the solution of the preventive or dipping animal teats in the solution of the preventive to form a coating having strong sterilizing activity onto the animal teats, sterilizing pathogenic microbes attached to the teats, and inhibiting the invasion of the pathogenic microbes into the udder of animals.

### Best Mode for Carrying Out the Invention

The present invention is now described further in detail with referring to the following examples, however, the scope of the present invention should not be limited to the description in those examples.

### Example 1

### [Evaluation Test for Antibacterial Activity]

The following MIC test was carried out for the sterilizing microbes listed in Table 1.

The antibacterial activity of the sterilizing microbe was evaluated by drug dilution agar plate method in accordance with the MIC evaluation method recommended by Japanese Society of Chemotherapy. The concentration of bacterial suspension was adjusted to a level of 10⁶ CFU/ml, incubated for 24 hours at 37°C, and assessed the fate of the bacteria. The results are shown in Table 1.
Bacteria tested; Staphylococcus aureus IFO 12732
Streptococcus agalactiae ATCC 13813
Escherichia coli ATCC 27166
Pseudomonas aenuginosa IFO 12689
sterilizing microbes tested;
Benzalkoniun chloride (BAC, Nissan Cation M₂-100, Nihon Yushi)
Benzethonium chloride (BEC, Hiamine, Sankyo)
Alkylglycine (AG, Nissan Anone LG, Nihon Yushi)
Chlorhexidine gluconate (CHG, Hibiden gluconate solution, ICI Pharma)
Iodohole (3% available iodine, Sanyo Kasei)
Chitosan (LLWP Kimitsu Kagaku)
The MIC values indicated in Table 1 means the concentration of the sterilizing microbe excluding chitosan. The composition ratio by weight of chitosan to the sterilizing microbe is 10 : 1.

Because of no compatibility between chitosan and iodohole, the evaluation on the activity of the combination of chitosan plus iodohole was not conducted.

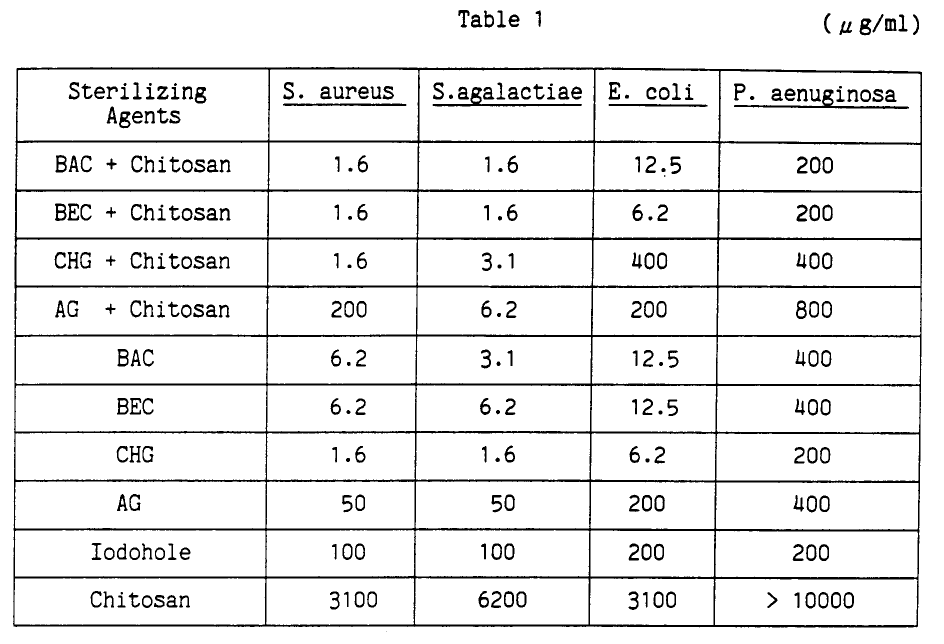

### Example 2

Each of test samples represented in Table 2 are prepared for Examples 1 and 2 and Comparative Examples 1 to 4, then the following tests were carried out.

### [Tests on Preventive Activity on Mastitis]

The test samples prepared according to the recipes represented in Examples 1 and 2 and Comparative Examples 1 to 4 were respectively sprayed to the teats of animals after milking for a period of 6 months.

The results are summarized in Table 3.

### [Test on the Skin Irritation Property of the Sterilizing Agent Composition]

Patch tests for each of the sterilizing microbe compositions prepared according to the recipes represented in Examples 1 and 2 and Comparative Examples 1 to 4 were conducted to each skin of the forearms of twenty male human adults by using lint cloths. The time for the treatment was 6 hours. After 24 hours from the completion of the treatment, the conditions of the skins tested were examined.

Also another observation was made during the process of this test on the condition of the skins. These results were summarized in Table 3.

**Table 2-1**

| Example 1 | | Example 2 | | Comparative Example 1 | |
|---|---|---|---|---|---|
| Components | Part By Weight (g) | Components | Part By Weight (g) | Components | Part By Weight (g) |
| Purified water | 100 | Purified water | 100 | Purified water | 100 |
| Glacial Acetic Acid | 0.5 | Lactic Acid | 1.5 | Glacial Acetic Acid | 0.5 |
| Chitosan (CTA-4) | 1.0 | Chitosan (L) | 1.0 | Chitosan (CTA-4) | 1.0 |
| Benzalkonium Chloride | 0.1 | Benzethonium Chloride | 0.1 | | |
| Ethanol | 5.0 | Ethanol | 5.0 | | |

**Table 2-2**

| Comparative Example 3 | | Comparative Example 4 | | Comparative Example 5 | |
|---|---|---|---|---|---|
| Components | Part By Weight (g) | Components | Part By Weight (g) | Components | Part By Weight (g) |
| Purified water | 100 | Purified water | 100 | Purified water | 100 |
| Glacial Acetic Acid | 0.5 | | | | |
| | | Popidon Iodine | 2.0 | | |
| Benzalkonium Chloride | 0.1 | | | | |
| Ethanol | 5.0 | | | | |

### Remarks;

1) CTA-4 : Chitosan manufactured by Katakura Tikkarin Co., Ltd.
2) L : Chitosan manufactured by Kimitsu Kasei Co., Ltd.
3) Nissan Cation M₂-100 (Manufactured by Nihon Yushi Co., Ltd.) was used for benzalkonium chloride.
4) Hiamine (Sankyo Co., Ltd.) was used for benzethonium chloride.
5) Popidon Iodine is polyvinylpyrrolidone Iodine (Manufactured by Fujita Seiyaku Co., Ltd.).

**Table 3**

| | Heads | No.of Outbreaks of Clinical Mastitis | Ratio (%) | Skin * Irritation (Human) | State of Teats of Dairy Cattle |
|---|---|---|---|---|---|
| Example 1 | 38 | 2 | 5.3 | - | Good |
| Example 2 | 39 | 2 | 5.1 | - | Good |
| Comparative Example 1 | 35 | 4 | 11.4 | - | Good |
| Comparative Example 2 | 35 | 3 | 8.6 | - | Good |
| Comparative Example 3 | 35 | 2 | 5.7 | +, ⁺⁺ | Cracks observed |
| Comparative Example 4 | 30 | 10 | 33.3 | - | Good |

| | | | | | |
|---|---|---|---|---|---|
| * Indications for Skin Irritation; | | | | | |
| - : No irritation. + : Erythema observed. ⁺⁺ : Edema and eruption observed. | | | | | |

### (Advantageous Effect)

As shown in the Test Example 1, the combining use of quaternary ammonium salt-containing sterilizing microbe and chitosan and/or the derivative thereof can maintain their sterilizing activities, and there is a case that this combination enhance the sterilizing activity in a synergistic manner. In the combination of other sterilizing microbes and chitosan, there is provided inferior results due to the deterioration in activity resulted from such combination. Furthermore, the combination of iodine-containing sterilizing microbes and chitosan were not compatible each other therefore, it was not preferable to use them in mixture.

As shown in the test on the preventive activity for mastitis in the Test Example 2 wherein dairy cattle were used, the preventive for mastitis of the present invention showed to have excellent preventive effect on mastitis comparing to the other reference microbes, while the iodine-containing preventive for mastitis produced cracks in the animal teats because of its skin irritating property.

By combining a specific sterilizing microbe and chitosan and/or the derivative thereof, the preventive for mastitis of the present invention has a characteristic that it can demonstrate excellent sterilizing activity without causing cracks in the animal teats because of less skin irritating property thereof and inhibit the invasion of the pathogenic microbes into the udder by forming a coating containing chitosan and/or the derivative thereof onto the animal teats.

It can be understood that such advantages of the preventive for mastitis of the present invention is derived from several factors, such as coating-forming property and sterilizing activity of chitosan and synergistic activity enhancement obtained from the combination of chitosan and quaternary ammonium salt-containing sterilizing microbe, those which provided a function for sterilizing animal teats and for preventing the invasion of pathogenic microbes into animal teats to the preventive of the invention.

### Industrial Applicability

As described above, it is an object of the present invention to provide a novel preventive for mastitis which forms a coating on the teats of livestock such as dairy cattle, prevents the propagation of the pathogenic microbes on the teats, and can inhibits the invasion of the pathogenic microbes into the udder, and an available means for preventing mastitis of livestock for dairy farmers.

## Claims

1. A preventive for mastitis of livestock characterized in that it comprises quaternary ammonium salt-containing sterilizing microbe and chitosan and/or the derivative thereof.

2. The preventive for mastitis according to Claim 1 wherein the quaternary ammonium salt-containing sterilizing microbe is benzalkonium chloride or benzethonium chloride.

3. The preventive for mastitis according to Claim 1 which contains the quaternary ammonium salt-containing sterilizing microbe in an amount of 2 to 50 parts by weight with respect to 100 parts by weight of chitosan and/or the derivative thereof.

4. The preventive for mastitis according to Claim 1 which is formed in an aqueous solution or aqueous solution of an organic acid or an inorganic acid, either containing the quaternary ammonium salt-containing sterilizing microbe and chitosan and/or the derivative thereof in an amount of 0.05% by weight to 10% by weight in total.
